**Europäisches Patentamt**

**European Patent Office**

**Office européen des brevets**

(19)

(11) Numéro de publication: **0 233 114**
**B1**

(12) **FASCICULE DE BREVET EUROPEEN**

(45) Date de publication du fascicule du brevet: **13.06.90**

(51) Int. Cl.⁵: **A 61 F 2/38**

(21) Numéro de dépôt: **87400179.5**

(22) Date de dépôt: **27.01.87**

(54) Composant tibial de prothèse unicompartimentaire du genou, à implanter sans ciment.

(30) Priorité: **27.01.86 FR 8601078**

(43) Date de publication de la demande:
**19.08.87 Bulletin 87/34**

(45) Mention de la délivrance du brevet:
**13.06.90 Bulletin 90/24**

(84) Etats contractants désignés:
**AT BE CH DE ES GB IT LI LU NL SE**

(56) Documents cités:
**EP-A-0 032 828**
**DE-A-3 429 157**
**FR-A-2 288 509**
**FR-A-2 290 883**
**FR-A-2 338 690**
**FR-A-2 378 505**
**FR-A-2 403 068**

(73) Titulaire: **Epinette, Jean-Alain**
**27 rue Lamandin**
**F-62700 Bruay-en-Artois (FR)**

(72) Inventeur: **Epinette, Jean-Alain**
**27 rue Lamandin**
**F-62700 Bruay-en-Artois (FR)**

(74) Mandataire: **CABINET BONNET-THIRION**
**95 Boulevard Beaumarchais**
**F-75003 Paris (FR)**

Courier Press, Leamington Spa, England.

**Description**

L'invention se rapporte à un composant tibial de prothèse unicompartimentaire de genou, comprenant une embase métallique en forme générale de segment de disque circulaire avec des faces généralement planes supérieure, inférieure et latérale, celle-ci perpendiculaire aux autres suivant la corde du segment, les faces inférieure et latérale étant prévues pour porter sur deux plans d'entaille pratiqués dans un plateau du tibia respectivement perpendiculairement à la direction générale du tibia et parallèlement à celle-ci en longeant le massif des épines, et un plateau en matière polymère solidarisé à la face supérieure de l'embase et formant garniture pour l'appui d'un composant condylien correspondant.

Dans l'articulation naturelle du genou existent trois paires articulaires, à savoir:

— une paire articulaire fémoro-tibiale interne où le condyle fémoral interne glisse sur le plateau tibial interne,

— une paire articulaire fémoro-tibiale externe où le condyle fémoral externe glisse sur le plateau tibial externe,

— une paire articulaire fémoro-patellaire, où la face postérieure de la rotule glisse sur la trochlée fémorale.

On peut classer les prothèses de genou en diverses catégories, suivant les paires articulaires remplac'ées:

— des prothèses qui comportent un articulation mécanique liant les composants fémoral et tibial, qui sont dites protèses totales contraintes ou semi-contraintes;

— des prothèses qui reproduisent les surfaces de contact des paires articulaires qui se divisent en:

— prothèses où sont remplacées les trois paires articulaires, dites prothèses totales à glissement;

— prothèses où est remplacée une paire fémoro-tibiale interne ou externe selon le cas, dites prothèses unicompartimentaires;

— prothèses où est remplacée la paire fémoropatellaire, dites prothèses de rotule.

On notera par ailleurs que, si l'on doit remplacer les deux paires fémoro-tibiales interne et externe, le composant tibial peut être unique. La présente invention se rapporte uniquement au composant tibial d'une prothèse unicompartimentaire.

Les composants tibiaux de prothèses unicompartimentaires viennent en coopération avec un composant condylien métallique en forme de croissant avec une surface qui épouse sensiblement un tore et qui est posé sur le condyle préparé par vissage ou scellement d'un téton avec un ciment acrylique dans une cavité pratiquée dans le condyle.

Les composants tibiaux courants de prothèses unicompartimentaires se composent d'une embase métallique généralement plane, en forme de segment de disque circulaire, avec un face inférieure où sont pratiquées des rainures destinées à faciliter l'accrochage d'un ciment acrylique. Sur l'embase est surmoulé un plateau en matériau polymère, typiquement un polyéthylène haute densité. Ce plateau comporte une face latérale plane qui prolonge une face correspondante de l'embase, suivant la corde du segment de cercle. Ces faces latérales viennent porter, après mise en place du composant, sur un mur taillé dans le flanc du massif des épines situé entre les deux plateaux tibiaux et sur lequel s'attachent les ligaments croisés du genou. Comme on l'a indiqué plus haut, la préparation du tibia comporte la resection du plateau par deux plans de sciage, l'un perpendiculaire à la direction générale du tibia et l'autre parallèle à cette direction en longeant le massif des épines, pour réaliser le mur évoqué ci-dessus.

Le composant est fixé dans l'entaille provoquée par la resection du plateau, par un ciment, généralement de type acrylique, la face inférieure de l'embase reposant à plat sur le plan de sciage perpendiculaire à la direction générale du tibia, tandis que la face latérale du composant porte sur le mur taillé dans le flanc du massif des épines.

Ce type de composant tibial présente les inconvénients classiques des prothèses cimentées, à savoir les risques de vieillissement du ciment polymérisé qui peut alors s'effriter ou se fissurer, ainsi que les risques d'une détérioration de l'os en contact avec le ciment acrylique vieilli, le résultat étant une prise de jeu du composant tibial. En outre il est nécessaire, lors de la pose de la prothèse, d'éviter de mettre un excès de ciment dans la région voisine de l'angle entre les plans de sciage, cet excès s'évacuant insuffisamment lorsque le composant est forcé dans l'entaille et risquant d'apporter un faux aplomb du plateau.

De plus, si l'usure du plateau de la prothèse nécessite un remplacement de ce plateau, on doit enlever tout le composant, reprendre les plans d'entaille du tibia et reposer un nouveau composant plus épais que le premier. Or l'épiphyse supérieur du tibia n'a qu'un volume limité, et il est fâcheux de l'entailler trop profondément.

On a proposé des composants tibiaux de prothèses unicompartimentaires à poser sans ciment. Ces prothèses sont constituées uniquement d'un plateau en matériau polymère. Le plateau est muni d'un pied cylindrique avec des ailettes circulaires étagées séparées par des gorges. Ces pieds peuvent être enfoncés à force dans des alésages pratiqués dans l'épiphyse tibiale dans la partie centrale du plan de sciage perpendiculaire à la direction générale du tibia. Ce type de composant présente lui aussi des inconvénients. La pose du composant exige le sacrifice d'une partie non négligeable de l'épiphyse, ce qui rend hasardeux un remplacement futur du composant. En outre l'alésage où s'enfonce le pied du composant est pratiqué dans un région où le tissu osseux est plus à l'état spongieux qu'à l'état corticoïde, et donc relativement tendre, ce qui est peu favorable à un maintien durable.

Aussi l'invention propose un composant tibial de prothèse unicompartimentaire du genou qui se pose sans ciment, ne nécessite qu'un sacrifice réduit d'os pour pratiquer l'entaille de pose, et dont le plateau en matériau polymère peut être désolidarisé de l'embase.

A cet effet l'invention propose un composant tibial de prothèse unicompartimentaire de genou, comprenant une embase métallique en forme générale de segment de disque circulaire avec des faces généralement planes, supérieure, inférieure et latérale, celle-ci perpendiculaire aux autres suivant la corde du segment, et une périphérie en arc de cylindre, les faces inférieure et latérale étant prévues pour porter sur deux plans d'entaille pratiqués dans le plateau du tibia, respectivement perpendiculairement à la direction générale du tibia, et parallèlement à celle-ci en longeant le massif des épines, et un plateau en matériau polymère solidarisé à la face supérieure de l'embase, et formant garniture pour l'appui d'un composant condylien correspondant, composant tibial destiné à être placé sans ciment et caractérisé en ce que l'embase présente, sur sa face latérale au moins une saillie apte à être enfoncée sous le massif des épines, au moins un passage pour un moyen de chevillage à engager dans l'os sous-jacent au voisinage de sa périphérie, et sur sa face supérieure des moyens de fixation du plateau utilisables après pose de l'embase, et en ce qu'au moins une partie de la surface de l'embase mise en contact avec l'os est garnie d'un revêtement de métal poreux apte à être envahi par de l'os spongieux en croissance.

Lors de la pose, l'embase est ancrée en place par la combinaison de la saillie qui est enfoncée sous le massif de épines et des moyens de chevillage engagés dans l'os sous-jacent à travers les passages voisins de la périphérie de l'embase. Cet ancrage primaire immédat est completé par la suite part l'envahissement du revêtement de métal poreux par de l'os spongieux en croissance qui constitue un scellement sans ciment. On notera que ce type de revêtement de métal poreux a été mis au point pour améliorer la tenue du ciment sur des prothèses articulaires, notamment prothèses de hanches, et s'est avéré par la suite apte à être scellé directement par croissance d'os spongieux, dans des conditions très supérieures à celles d'un scellement classique au ciment.

Par ailleurs la pose de l'embase n'est pas gênée par la présence du plateau, que l'on vient solidariser par la suite. Et il est clair que s'il apparaissait une usure du plateau, ou une imperfection d'adaptation, il serait possible de changer le plateau sans toucher à la solidarisation entre l'embase et le tibia.

De préférence la saillie de la face latérale est une lame trapézoïdale parallèle aux plans des faces supérieure et inférieure en prolongeant celle-ci, la grande base de la lame se confondant avec la face latérale. Cette forme assure un ancrage immédiat avec un maximum de résistance aux efforts normaux à l'embase, susceptibles de provoquer de faux aplombs.

De préférence encore cette lame comporte au moins un orifice et le revêtement de métal poreux s'étend sur la totalité des deux faces de la lame. Cette disposition assure à terme un scellement complet de la lame dans le massif des épines, avec formation de ponts osseux à travers les orifices.

De même on préfère que la totalité des faces de l'embase en appui sur de l'os soit garnie du revêtement de métal poreux, afin de bénéficier d'un scellement de surface maximale.

En disposition préférée le plateau présente une surface supérieure à concavité à grand rayon, ce qui est favorable à un appui précis du composant condylien.

En disposition préférée les moyens de fixation du plateau comprennent un tenon à profil en queue d'aronde saillant de la face supérieure de l'embase et s'étendant généralement parallèlement à la face latérale, et une mortaise complémentaire engagée dans le plateau. Il est préférable que le tenon ait la forme d'un coin s'évasant d'une tranche ayant à une tranche arrière, de sorte que l'engagement de la mortaise parallèlement à la face latérale amène un coincement du tenon. La sécurité de mise en place est encore améliorée en munissant l'embase d'un rebord qui fait saillie de la face supérieur en prolongement de la périphérie cylindrique et de la face latérale. Ainsi l'emboîtement du plateau dans la cavité limitée par ce rebord, grâce à l'élasticité du matériau polymère empêche tout mouvement du plateau par rapport à l'embase sous l'effet des contraintes de l'articulation.

Il ests préférable qu'une pluralité de passages pour moyens de chevillage soient ménagés symétriquement par rapport au plan médiateur de la face latérale, afin que la portée de l'embase sur l'os sous-jacent préparé soit équilibrée et corresponde à une transmission des efforts à travers l'articulation qui emprunte le trajet naturel des lignes de forces.

Les moyens de chevillage sont de préférence des vis dont la tête est noyée dans des fraisages pour ne pas contrarier l'appui du plateau sur la face supérieure de l'embase.

D'autres caractéristiques et avantages de l'invention ressortiront d'ailleurs de la description qui va suivre à titre d'exemple, en référence aux dessins annexés dans lesquels:

la figure 1 est une perspective d'ensemble d'un composant tibial de prothèse du genou selon l'invention;

la figure 2 est une vue de dessus d'une embase de composant tibial;

la figure 3 est une coupe suivant le plan III—III de la figure 2;

la figure 4 est une vue de dessus d'un plateau de composant tibial;

la figure 5 est une coupe suivant le plan V—V de la figure 4;

la figure 6 est une vue schématique, partiellement coupée d'une porthèse unicompartimentaire interne du genou comprenant un composant tibial de l'invention.

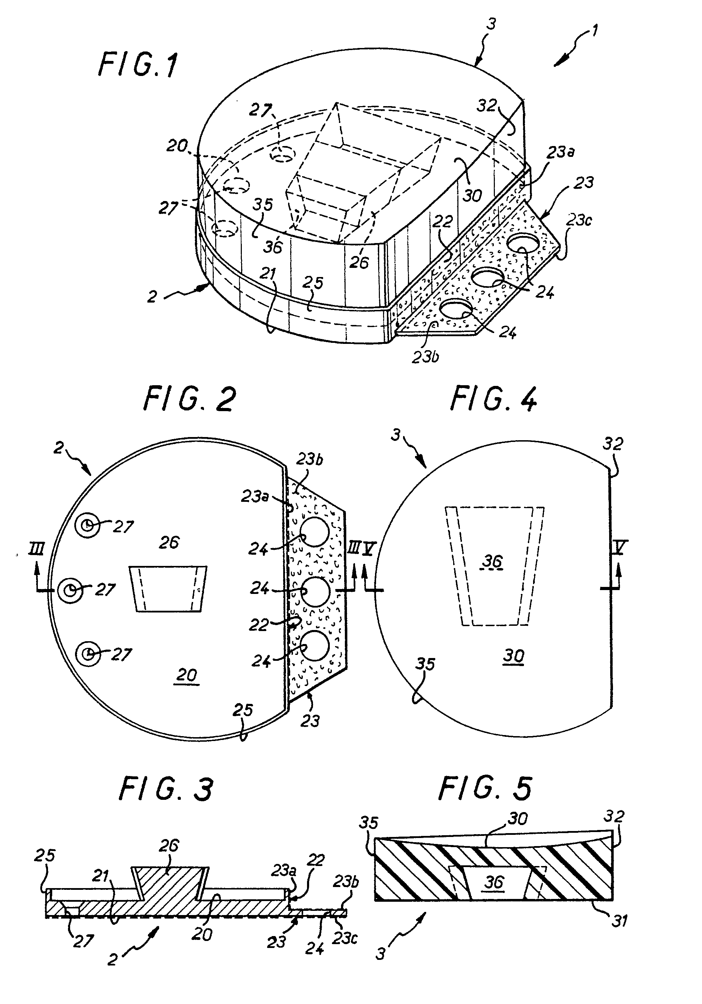

Selon la forme de réalisation choisie et représentée figures 1 à 5, un composant tibial 1 dans son ensemble comprend une embase métallique 2 en acier inoxydable ou en titane TA6V, et un plateau 3, en matière polymère, ici un polyéthylène haute densité de qualité biocompatible RCH 1 000.

L'embase 2 est en formé générale de segment de disque circulaire avec une face supérieure 20, une face inférieure 21 et une face latérale 22, ces trois faces étant généralement planes et la face latérale 22, étendue suivant la corde de segment circulaire, étant perpendiculaire aux faces 20, 21. La face supérieure 20 est limitée par un rebord 25 qui s'étend dans le prolongement de la périphérie du disque et de la face latérale 22. Sensiblement au centre de la face 20 fait saillie un tenon 26 à profil en queue d'aronde et disposé en coin dans un sens longitudinal parallèle à la face latérale 22. Dans l'épaisseur de l'embase, en partant de la face 20 et à proximité de la périphérie circulaire du segment sont pratiqués des passages 27, avec une fraisure tronconique adaptée à noyer des têtes fraisées de vis. Ces passages, ici au nombre de trois, sont disposés en symétrie par rapport à un plan médiateur de la face latérale 22, c'est-à-dire un passage dans ce plan médiateur et les deux autres symétriques par rapport à ce plan.

Faisant saillie de la face latérale 22, une lame 23 s'étend dans le prolongement de la face inférieure 21. Cette lame est en forme de trapèze isocèle avec sa grande base 23a confondue avec la face latérale 22, et comporte trois ouvertures circulaires 24 dont les centres sont alignés parallèlement aux bases du trapèze, à égales distances de ces bases.

La face inférieure 21, la face latérale 22 et les deux faces 23b et 23c de la lame 23 sont garnies d'un revêtement de métal poreux, formé de particules projetées par exemple à la torche à plasma. Ce type de revêtement, conçu à l'origine pour ameliorer la tenue des ciments acryliques sur les pièces métalliques de prothèses articulaires, s'est avéré apte à être envahi par de l'os spongieux en croissance, et à procurer ainsi après un délai postopératoire qui se compte en semaines, un excellent ancrage de pièces métallique sur les os.

Le plateau en polyéthylène 3 présente un forme générale de segment de disque avec une face inférieure 31 plane, une face latérale 32, et une périphérie cylindrique 35, aptes à se loger à l'intérieur du rebord 25 avec la face inférieure 31 en appui sur la face supérieure 20 de l'embase.

La face supérieure 30 a la forme d'une calotte sphérique, concave, à grand rayon de courbure, pour améliorer, comme on le verra plus loin, l'appui d'un composant condylien.

La face inférieure 31 présente une mortaise 36 à profil en queue d'aronde, complémentaire du tenon 26 de l'embase 2, et présenté également longitudinalement une forme de coin. La mortaise 36 est prévue telle que le tenon 26 s'y bloque lorsque le plateau 3 est en position pour s'emboîter dans le rebord 25, et présente, du côté où cette mortaise s'évase, une longueur suffisante pour que le tenon 26 pénètre dans la mortaise 36 verticalement, les arêtes du tenon passant entre les arêtes de la mortaise.

On comprendra que, pour mettre en place le plateau 3 sur l'embase 2, il faut forcer élastiquement le plateau contre l'embase, le plateau 3 portant sur le rebord 25, et que l'emboîtement à l'intérieur du rebord 25 nécessite d'insérer une lame entre rebord 25 et périphérie 35 du plateau 3 et de pousser la lame en levier en relevant la lame.

Par ailleurs, la mise en place du plateau sur l'embase résulte d'un glissement parallèle à la face latérale 22 de l'embase, du sommet à la base des coins en queue d'aronde 26 et 36.

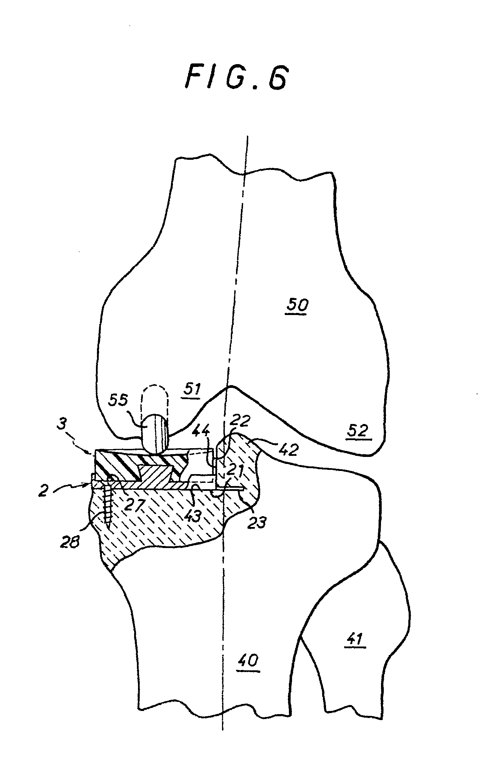

Pour mieux comprendre les raisons pour lesquelles les éléments du composant tibial 1 ont les structures décrites, on se référera à la figure 6.

L'articulation du genou ici gauche est représentée de face, avec mise en place d'une prothèse unicompartimentaire interne.

Sur le tibia 40 s'articule latéralement, du côté externe, le péroné 41. Au-dessus le fémur 50 se termine par les condyles interne 51 et externe 52. Entre les condyles se situe une échancrure qui coiffe le massif des épines 42 entre les plateaux du tibia. Sur le dessin la rotule n'est pas représentée; au stade représenté de pose de la prothèse la rotule est luxée et repoussée pour dégager l'articulation.

Sur le condyle interne 51 a été posé un composant condylien 55, qui se présente comme une fraction de tore, en sorte de présenter une surface convexe arrondie transversalement en gouttière, et perpendiculairement au plan au dessin, une configuration en croissant qui épouse la courbure du condyle. L'arête, arrondie, du composant condylien 55 doit glisser sur le plateau tibial.

La préparation du tibia, ici tibia gauche, pour pose d'une protèse unicompartimentaire, ici interne, comporte la resection du plateau naturel par deux plans d'entaille, un plan d'entaille 43 perpendiculaire à la direction générale du tibia (en traits mixtes) et l'autre 44 parallèle à cette direction générale du tibia et longeant le massif des épines 42 (perpendiculairement donc au plan du dessin).

L'embase 2 est posée sur le plan d'entaille 43 et poussée latéralement (dans une direction parallèle au plan de la figure) en forçant la lame 23 dans l'os spongieux sous le massif des épines 43, jusqu'à ce que la face latérale 22 de l'embase porte sur le plan d'entaille 44.

Après cela on fore, à travers les passages 27, des logements pour des vis 28 formant moyens de chevillage, qui sont ensuite vissées dans l'os, parallèlement à la direction générale du tibia 40 jusqu'à noyer les têtes dans les fraisages tronconiques, tandis que la face inférieure 21 de l'embase 2 repose bien à plat sur le plan de coupe 43.

Après cela, on choisit un plateau 3 d'épaisseur convenable pour reconstituer l'appui naturel de l'articulation, et on l'engage sur le tenon, parallèlement au plan de coupe 44, comme on l'a déjà expliqué.

On appréciera que, de ce moment, le composant tibial est ancré à son emplacement définitif, sans risque d'une perte d'aplomb du composant sur le tibia. Mais cet ancrage primaire, qui pourrait éventuellement se détériorer à la longue, se trouve consolidé par un ancrage secondaire résultant de l'envahissement du revêtement des faces inférieure et latérale de l'embase 2 et les deux faces de la lame 25, soit par toutes les surfaces de l'embase au contact de l'os, par l'os spongieux en croissance. En outre la croissance de l'os conduira à la formation, à travers les ouvertures 24 de la lame 23 de ponts osseux qui interdisent un arrachement de cette lame.

On appréciera de plus que, si éventuellement la prothèse donnait, au fil des ans, des signes d'usure (usure de plateau de polyéthylène haute densité), il reste possible de changer le plateau sans avoir à supprimer l'ancrage de l'embase, et à accentuer en conséquence le sacrifice d'os dans le plateau du tibia.

On aura compris que si la description donne des informations sur la méthode de pose des composants tibiaux de l'invention, c'est uniquement pour faire ressortir les particularités structurelles de ces composants et la motivation de leur adoption. Il est clair que la méthode de pose est en dehors du cadre de l'invention.

Bien entendu l'invention n'est par limitée à ce qui est décrit à titre d'exemple, et embrasse toutes les variantes d'exécution dans la cadre des revendications.

En particulier l'orientation du petit côté de l'assemblage à queue d'aronde 26—36 sera déterminée, suivant que le composant est gauche ou droit, interne ou externe, pour que la mise en place du plateau se fasse par l'avant du genou (emplacement de la rotule) seul accessible pendant l'intervention.

Notamment le tenon 26, au lieu d'avoir une forme de coin, pourrait être réalisé en forme de tronc de cône d'axe perpendiculaire à la face supérieure 20, se confondant par sa petite base avec cette face supérieure 20.

Egalement on pourrait envisager, bien qu'actuellement les vis constituent les moyens de chevillage préférables, que les vis soient remplacées par des chevilles bloquées par expansion, ou des agrafes.

## Revendications

1. Composant tibial (1) de prothèse unicompartimentaire de genou, comprenant une embase (2) métallique en forme générale de segment de disque circulaire avec des faces généralement planes, supérieur (20), inférieure (21) et latérale (22), celle-ci perpendiculaire aux autres suivant la corde du segment, et une périphérie en arc de cylindre, les faces inférieure (21) et latérale (22) étant prévues pour porter sur deux plans d'entaille (43, 44) pratiqués dans le plateau du tibia (40), respectivement perpendiculairement à la direction générale du tibia (40), et parallèlement à celle-ci en longeant le massif des épines (42), et un plateau (3) en matériau polymère solidarisé à la face supérieure (20) de l'embase, et formant garniture pour l'appui d'un composant condylien (55) correspondant, composant tibial destiné à être placé sans ciment et caractérisé en ce que l'embase (2) présente, sur sa face latérale (22), au moins une saillie (23) apte à être enfoncée sous le massif des épines (42), au moins un passage (27) pour un moyen de chevillage (28) à engager dans l'os sous-jacent au voisinage de sa périphérie, et sur sa face supérieur (20) des moyens de fixation (26) du plateau (3) utilisables après pose de l'embase (2), et en ce qu'au moins une partie de la surface de l'embase (2) mise en contact avec l'os est garnie d'un revêtement de métal poreux apte à être envahi par de l'os spongieux en croissance.

2. Composant tibial selon la revendication 1, caractérisé en ce que la saillie (23) de la face latérale (22) est une lame trapézoïdale isocèle en prolongement de la face inférieure (21), la grande base (23a) de la lame se confondant avec la face latérale (22).

3. Composant tibial selon la revendication 2, caractérisé en ce que ladite lame (23) présente au moins un orifice (24), le revêtement de métal poreux s'étendant sur la totalité des faces (23b, 23c) de cette lame.

4. Composant tibial selon la revendication 1 à 3, caractérisé en ce que la totalité des faces (21, 22) de l'embase (2) prévues pour l'appui sur l'os est garnie dudit revêtement de métal poreux.

5. Composant tibial selon une quelconque des revendications 1 à 4, caractérisé en ce que ledit plateau (3) présente une face supérieure (30) à concavité à grand rayon de courbure.

6. Composant tibial selon une quelconque des revendications 1 à 5, caractérisé en ce que le moyen de fixation (26) du plateau sur l'embase (2) comprend un tenon à profil en queue d'aronde saillant de la face supérieure (20) de l'embase et s'étendant généralement parallèlement à la face latérale (22), le plateau (3) comportant dans sa face inférieure (31) une mortaise (36) complémentaire, suivie d'un dégagement pour le tenon.

7. Composant tibial selon la revendication 6, caractérisé en ce que le tenon (26) est en forme de coin s'évasant d'une tranche avant à une tranche arrière suivant le sens longitudinal, la mortaise (36) se prolongeant dans le sens de l'évasement suffisamment pour dégager le tenon (26).

8. Composant tibial selon une quelconque des revendications 1 à 7, caractérisé en ce qu'un rebord (25) fait saille de la face supérieure (20) de l'embase (2) en prolongement de la périphérie cylindrique et de la face latérale (22), le plateau (3) étant apte à s'emboîter dans le rebord (25).

9. Composant tibial selon une quelconque des revendications 1 à 8, caractérisé en ce que l'embase (2) comporte une pluralité de passages (27) pour des moyens de chevillage (28), répartis symétriquement par rapport à un plan médiateur de la face latérale (22).

10. Composant tibial selon une quelconque des revendications 1 à 9, caractérisé en ce que les moyens de chevillage sont des vis (28) à tête, les passages (27) comportant des fraisages propres à noyer les têtes de vis (28).

## Patentansprüche

1. Zementfrei implantierbarer Tibiateil einer einseitigen Kniegelenk-Endoprothese mit einer Grundplatte (2) aus Metall in der allgemeinen Form eines Kreisscheibensegments mit im ganzen ebenen Flächen, einer oberen (20), unteren (21) und einer seitlichen (22), die senkrecht zu den anderen gemäß der Sehne des Segments verläuft, und mit einem Umfang in Form eines Zylinderbogens, wobei die untere (21) und seitliche Fläche (22) dafür vorgesehen sind, sich auf zwei Schnittflächen (43, 44) abzustützen, die im Plateau der Tibia (40) jeweils senkrecht zur Gesamtrichtung der Tibia (40) und parallel zu dieser längs der Eminentia intercondylica (42) ausgebildet sind und mit einer Platte (3) aus Kunststoffmaterial, die fest mit der oberen Fläche (20) der Grundplatte verbunden ist und einen Belag für die Auflage eines entsprechenden Condylenteils (55) bildet, dadurch gekennzeichnet, daß die Grundplatte (2) an ihrer seitlichen Fläche (22) mindestens einen Vorsprung (23) aufweist, der zum Einpressen unter die Eminentia intercondylica (42) geeignet ist, in de Nähe ihres Umfanges mindestens einen Durchlaß (27) für eine Verdübelungsvorrichtung (28), di zum Eingriff in den darunterliegenden Knochen bestimmt ist, und auf iher oberen Fläche (20) eine Befestigungsvorrichtung (26) für die Platte (3) aufweist, die nach dem Einsetzen der Grundplatte (2) benutzbar ist, und daß mindestens ein Teil der mit dem Knochen in Berührung kommenden Fläche der Grundplatte (2) mit einem Überzug aus porösem Metall versehen ist, in den der spongiöse Knochen hineinwachsen kann.

2. Tibiateil nach Anspruch 1, dadurch gekennzeichnet, daß der Vorsprung (23) der seitlichen Fläche (22) ein die untere Fläche (21) fortsetzendes Blatt in Gestalt eines gleichschenkeligen Trapezes ist, dessen große Basis (23a) mit der seitlichen Fläche (22) zusammenfällt.

3. Tibiateil nach Anspruch 2, dadurch gekennzeichnet, daß das Blatt (23) mindestens eine Öffnung (24) aufweist und der Belag aus porösem Metall sich über die Gesamtheit der Flächen (23b, 23c) dieses Blattes erstreckt.

4. Tibiateil nach einem der Ansprüche 1 bis 3, dadurch gekennzeichnet, daß alle Flächen (21, 22) der Grundplatte (2), die zum Aufliegen auf dem Knochen vorgesehen sind, mit dem Belag aus porösem Metall versehen sind.

5. Tibiateil nach einem der Ansprüche 1 bis 4, dadurch gekennzeichnet, daß die Platte (3) eine obere Fläche (30) mit einer Einwölbung mit großem Krümmungsradius aufweist.

6. Tibiateil nach einem der Ansprüche 1 bis 5, dadurch gekennzeichnet, daß die Befestigungsvorrichtung (26) der Platte (3) auf der Grundplatte (2) einen Zapfen mit Schwalbenschwanzprofil aufweist, der von der oberen Fläche (20) der Grundplatte vorspringt und sich im ganzen parallel zur stielichen Fläche (22) erstreckt, und die Platte (3) an inhrer unteren Fläche (31) ein komplementäres Zapfenloch (36) und anschließend einen Freiraum für die Freigabe des Zapfens aufweist.

7. Tibiateil nach Anspruch 6, dadurch gekennzeichnet, daß der Zapfen (26) keilförmig ist und sich von einer vorderen Schnittfläche zu einer in der Längsrichtung folgenden hinteren Schnittfläche verbreitert und daß das Zapfenloch (36) sich in Richtung der Verbreiterung genügend weit fortsetzt, um den Zapfen (26) freizugeben.

8. Tibiateil nach einem der Ansprüche 1 bis 7, dadurch gekennzeichnet, daß eine Randleiste (25) von der oberen Fläche (20) der Grundplatte (2) in Verlängerung des zylindrischen Umfangs vorspringt und die seitliche Fläche (22) der Platte (3) so ausgebildet ist, daß sie sich in die Randleiste (25) einfügen kann.

9. Tibiateil nach einem der Ansprüche 1 bis 8, dadurch gekennzeichnet, daß die Grundplatte (2) eine Mehrzahl von Druchlaßöffnungen (27) für Verdübelungsvorrichtungen (28) aufweist, die symmetrisch bezüglich einer mittelsenkrechten Ebene der seitlichen Fläche (22) verteilt sind.

10. Tibiateil nach einem der Ansprüche 1 bis 9, dadurch gekennzeichnet, daß die Verdübelungsvorrichtungen Flachkopfschrauben (28) sind und die Durchlaßoffnungen (27) geeignete Ausfräsungen zur Aufnahme der Köpfe der Schrauben (28) aufweisen.

## Claims

1. A tibial component (1) for a unicompartmental knee prosthesis, comprising a metal base (2) in the general form of a segment of a circular disc with a generally flat upper face (20), a generally flat lower face (21) and a generally flat lateral face (22), the last-mentioned face being perpendicular to the other faces along the chord of the segment, and comprising a cylindrical arc periphery, the lower and lateral faces (21) and (22) being adapted to bear on two notch planes (43, 44) provided in the plate of the tibia (40) respectively perpendicularly to the general direction of the tibia (40) and parallel thereto by extension along the condylar prominence (42), and a plate (3) of polymer material being firmly secured to the upper face (20) of the base and forming a lining for supporting a corresponding condylar component (55), said tibial component being intended for cementless implantation and characterised in that on its lateral face (22) the base (2) has at least one projection (23) designed to be driven under the condylar prominence (42), at least one passage (27) for a pinning means (28) to be engaged in the subjacent bone in the vicinity of its periphery, and on its upper face (20) fastening means (26) for the plate (3) which can be used after the base (2) has been fitted, and in that at least one part of the surface of the base (2) brought into contact with the bone is provided with a porous metal covering able to be invaded by growing spongy bone.

2. A tibial component according to claim 1, characterised in that the projection (23) of the lateral face (22) is an isosceles trapezium-shaped laminar member forming an extension of the inner face (21), the major base (23a) of the laminar member merging into the lateral face (22).

3. A tibial component according to claim 2, characterised in that said laminar member has at least one opening (24), the porous metal covering extending over the entirety of the faces (23b, 23c) of said laminar member.

4. A tibial component according to any one of claims 1 to 3, characterised in that the entirety of the faces (21, 22) of the base (2) provided to bear on the bone is provided with said porous metal covering.

5. A tibial component according to any one of claims 1 to 4, characterised in that the plate (3) has an upper face (30) with a concavity having a large radius of curvature.

6. A tibial component according to any one of claims 1 to 5, characterised in that the means (26) for fastening the plate to the base (2) comprises a tenon of dovetail section projecting from the upper face (20) of the base and extending substantially parallel to the lateral face (22), the lower face (31) of the plate (3) comprising a complementary mortise (32), and means for releasing the tenon.

7. A tibial component according to claim 6, characterised in that the tenon (26) is wedge- shaped and is flared from a front edge to a rear edge in longitudinal direction, the mortise (36) being extended sufficiently in the direction of the flared portion to enable the tenon (26) to be released.

8. A tibial component according to any one of claims 1 to 7, characterised in that a rim (25) projects from the upper face (20) of the base (2), thereby extending the cylindrical periphery and the lateral face (22), the plate (3) being designed to fit into the rim (25).

9. A tibial component according to any one of claims 1 to 8, characterised in that the base (2) comprises a plurality of passages (27) for pinning means (28) which are symmetrically distributed relative to an intermediate plane of the lateral face (22).

10. A tibial component according to any one of claims 1 to 9, characterised in that the pinning means are screws (28) with heads, the passages (27) being countersunk in such a way that the heads of the screws (28) are received flush.

EP 0 233 114 B1

FIG.1

FIG. 2

FIG. 4

FIG. 3

FIG. 5

1

# FIG. 6